# EUROPEAN PATENT APPLICATION

(11) **EP 3 305 405 A1**
(43) Date of publication of application: **11.04.2018**
(21) Application number: 16306337.3
(22) Date of filing: 10.10.2016
(51) Int. Cl.: B01J 23/889, B01J 23/34, B01J 23/656, B01J 23/68, B01J 37/03, B01J 37/12, C07C 45/51, C07C 45/52, C07C 45/38, C07C 45/39, C07D 213/48, C07D 307/12, C07D 213/30, C07D 307/42, B01J 37/14, B01J 35/00, B01J 35/02

(54) **MANGAN(III)-MANGAN(IV) MIXED OXIDE CATALYST CONTAINING AN INTERCALATED CATION X FOR AEROBIC OXIDATIVE CLEAVAGE OF 1,2-DIOLS**

(71) Applicant: Centre National de la Recherche Scientifique, 75794 Paris Cedex 16 (FR); Yale University, New Haven, CT 06510 (US); UNIVERSITE DE MONTPELLIER, 34090 Montpellier (FR)
(72) Inventor: ESCANDE, Vincent, 30133 LES ANGLES (FR); GRISON, Claude, 34170 CASTELNAU-LE-LEZ (FR); ANASTAS, Paul, GUILFORD, CT Connecticut CT 06437 (US)
(74) Representative: Bourgouin, André

(57) **Abstract**

The present invention is directed to a manganese-X mixed oxide, its use as a catalyst in oxidation reactions and a process for preparing said manganese-X mixed oxide.

In the manganese-X mixed oxide manganese is at the oxidation state +III and +IV and X is a cation selected from the group consisting of sodium, calcium, magnesium, cesium, lithium, potassium, iron, zinc, strontium, cobalt, cerium, vanadium, molybdenum, tin, tungsten, titanium, chromium, nickel, copper, ruthenium, bismuth, antimony, aluminum, silver, rhenium, osmium, rhodium, iridium, palladium, gold and tetramethylammonium.

Use of the manganese-X mixed oxide for oxidative cleavage of a 1,2-diol; a 1,2-diol monoether; a 1,2-diamine; a 1,2-aminoalcohol; an a-hydroxy-carbonyl compound, in particular selected from an a-hydroxy-acid or a derivative thereof such as an a-hydroxy-ester, an a-hydroxy-ketone and an a-hydroxy-aldehyde; an a-dicarbonyl compound such as an a-ketoaldehyde, a-diketone or glyoxal; or an epoxide; preferably a 1,2-diol or an a-hydroxy-acid is described.

## Description

The present invention is directed to a manganese-X mixed oxide, its use as a catalyst in oxidation reactions and a process for preparing said manganese-X mixed oxide.

Oxidative cleavage of 1,2 diols (also called glycols) has found uncountable applications in synthetic organic chemistry. This reaction leads to the fission of carbon-carbon bonds, producing aldehydes or ketones. Since its discovery in the early 20th century, this reaction is still performed nowadays by using the same archetypal reagents, mainly periodic acid and its salts or lead tetraacetate.

In spite of their high efficiency, these reagents are associated with a lot of drawbacks, as they are very toxic for human health and environment or can lead to explosions and alternative catalysts are highly desirable.

In a first aspect, the present invention concerns the use of a manganese-X mixed oxide, for the oxidative cleavage of a 1,2-diol; a 1,2-diol monoether, a 1,2-diamine; a 1,2-aminoalcohol; an α-hydroxy-carbonyl compound, an α-dicarbonyl compound or an epoxide.

In a second aspect, the present invention concerns a process for preparing a manganese-X mixed oxide having a higher activity as oxidation catalyst, in particular the oxidative cleavage of a 1,2-diol; a 1,2-diol monoether, a 1,2-diamine; a 1,2-aminoalcohol; an α-hydroxy-carbonyl compound, an α-dicarbonyl compound or an epoxide than the ones known in prior art.

In a third aspect, the present invention concerns the manganese-X mixed oxide prepared according to the process of the invention as catalyst in oxidation reactions, in particular the oxidative cleavage of 1,2-diol; a 1,2-diol monoether, a 1,2-diamine; a 1,2-aminoalcohol; an α-hydroxy-carbonyl compound, an α-dicarbonyl compound or an epoxide.

The benefits of the present invention can be summarized as follows:
- the catalysts are based on a non-toxic and cheap metal (manganese),
- they are more active than commercial MnO₂, activated or not, in particular in oxidative cleavage of 1,2-diols, and can hence be used in substoechiometric amounts, of some ppms,
- they are highly active with environmentally benign oxidants such as O₂ or simply air., can be used in catalytic amount and with poor oxidant such as dioxygen or air from the atmosphere,
- the catalyst can be recycled by simple filtration.

All these properties of the catalyst make it a valuable tool for green organic synthesis.

According to a first aspect, the present invention concerns the use of a manganese-X mixed oxide, wherein the manganese is at the oxidation state +III and +IV and X is a cation selected from the group consisting of sodium, calcium, magnesium, cesium, lithium, potassium, iron, zinc, strontium, cobalt, cerium, vanadium, molybdenum, tin, tungsten, titanium, chromium, nickel, copper, ruthenium, bismuth, antimony, aluminum, silver, rhenium, osmium, rhodium, iridium, palladium, gold and tetramethylammonium for the oxidative cleavage of a 1,2-diol; a 1,2-diol monoether, a 1,2-diamine; a 1,2-aminoalcohol; an α-hydroxy-carbonyl compound, in particular selected from an α-hydroxy-acid or a derivative thereof such as an α-hydroxy-ester, an α-hydroxy-ketone and an α-hydroxy-aldehyde; an α-dicarbonyl compound such as an α-ketoaldehyde, α-diketone or glyoxal; or an epoxide; preferably a 1,2-diol or an α-hydroxy-acid.

These manganese oxides are known and have been described for example in Handbook of Layered Materials (2004, Ed: M. Dekker), in particular in chapter 9 thereof.

Layered manganese oxides can be classified into three main types of structures:
(1) feitknechtite and pyrochroite, which have the Cdl₂ type of structure;
(2) birnessite and buserite type of materials (OL-1), in which inorganic/organic cations and water molecules are introduced into the interlayer region; and
(3) pillared layered manganese-based materials (POL) and manganese-based mesoporous materials (MOMS)/manganese-based porous mixed oxides (MANPOs).

Said Manganese-X mixed oxide can be a natural or synthetic birnessite or buserite. Birnessite (Na₄Mn₁₄O₂₇.7H₂O, JCPDS 32-1128) and buserite (Na₄Mn₁₄O₂₇.7H₂O, JCPDS 23-1046) are two naturally occurring materials, existing as the primary manganese oxide phase in deep-sea nodules. They consist of manganese oxide sheets with exchangeable hydrated cations between these sheets. Each of the manganese oxide layers is made up of edge-shared MnO₆ octahedral units. The manganese in these layered manganese oxide materials is mixed valent, with Mn⁴⁺, Mn³⁺ and Mn²⁺. Buserite is the initial form of birnessite before dehydration. Natural birnessite is poorly crystalline and impure in composition. Synthetic birnessites and buserites have also been prepared.

In the sense of the present invention, a Manganese-X oxide is defined as a material having a particulate structure containing manganese at the oxidation state (III), *i.e*. in the form of Mn₂O₃; at the oxidation state (IV), *i.e*. MnO₂; and optionally at the oxidation state (II), *i.e*. for example as MnO and at least one cation within the structure. In an embodiment, the manganese in said Manganese-X mixed oxide is composed of between about 20 and about 40 mol% of Mn(III) and between 60 and 80 mol% of Mn(IV). In particular, the Manganese-X mixed oxide is composed of about 30 mol% of Mn(III) and about 70 mol% of Mn(IV), based on the total Manganese present, as determined by mineral analysis and thermogravimetric analysis as described in the examples. Said manganese hence has a charge of + 3,7 (according to J. AM. CHEM. SOC. 2008, 130, 15938-15943).

In the present invention, the cation is selected from the group consisting of sodium, calcium, magnesium, cesium, lithium, potassium, iron, zinc, strontium, cobalt, cerium, vanadium, molybdenum, tin, tungsten, titanium, chromium, nickel, copper, ruthenium, bismuth, antimony, aluminum, silver, rhenium, osmium, rhodium, iridium, palladium, gold and tetramethylammonium.

In an advantageous embodiment, said cation is selected from the group consisting of sodium, calcium, magnesium, cesium, lithium, potassium, iron, tetramethylammonium, strontium and zinc.

In a more advantageous embodiment, the cation is selected from the group consisting of sodium, calcium, strontium and zinc.

Preferably, the cation is sodium. The invention hence concerns the use of a manganese-X mixed oxide, wherein the manganese is at the oxidation state +III and +IV and X is sodium, for the oxidative cleavage of a 1,2-diol; a 1,2-diol monoether, a 1,2-diamine; a 1,2-aminoalcohol; an α-hydroxy-carbonyl compound, in particular selected from an α-hydroxy-acid or a derivative thereof such as an α-hydroxy-ester, an α-hydroxy-ketone and an α-hydroxy-aldehyde; an α-dicarbonyl compound such as an α-ketoaldehyde, α-diketone or glyoxal; or an epoxide; preferably a 1,2-diol or an α-hydroxy-acid.

In an advantageous embodiment, the manganese mixed oxide has a layered structure. The Manganese-X mixed oxide is therefore advantageously not a tunnel type structure as found for example in pyrolusite, cryptomelane or todorokite.

The Manganese oxides according to the present invention are in the form of water insoluble particles, in particular having a diameter of 1 nm to 100 µm, notably 150 to 500 nm, in particular 50 to 150 nm. The particles can exist in the form of discrete particles having diameter of about 1 nm to 1000 nm, in particular 50 to 500 nm or in the form of aggregates of 1 to several µm.

In one more advantageous embodiment, the manganese-X mixed oxide has a layered structure and is in amorphous state (*i.e*. it is not crystalline). Without wishing to being bound by theory, it is speculated that layered Manganese-X mixed oxide in the amorphous state are more active in the oxidative cleavage reactions than their crystalline counterparts.

In one particular embodiment, the Manganese-X mixed oxide used for the oxidative cleavage reactions is devoid of Manganese (II) as determined by *X-Ray photoelectron spectrometry* (XPS). In that case, the spectrum of the Manganese-X mixed oxide is devoid of the specific Mn2p peaks at a value lower than 641 eV and a satellite peak at about 647 eV (Geochimica et Cosmochimica Acta Vol. 58, No. 22, pp. 4985-4999. 1994). Because Mn(II) is not active as a catalyst for oxidative cleavage, the Manganese-X mixed oxide according to the present invention only contains Manganese in an active form which may contribute to the enhanced observed.In an advantageous embodiment, the present invention concerns the use of a Manganese-X mixed oxide as defined above for the oxidative cleavage of a 1,2-diol; a 1,2-diol monoether, a 1,2-diamine; a 1,2-aminoalcohol; an α-hydroxy-carbonyl compound; an α-dicarbonyl compound such as an α-ketoaldehyde, α-diketone or glyoxal; or an epoxide; wherein:
- at least one of the hydroxyl groups, in particular the two hydroxyl groups of the diol, is adjacent to a multiple bond, such as a double or a triple bond, an aromatic ring or a heteroaromatic ring,
- at least one of the amino groups of the 1,2-diamine, in particular the two amino groups of the diamine, is adjacent to a multiple bond, such as a double or a triple bond, an aromatic ring or a heteroaromatic ring,
- the amino group or the hydroxyl group, in particular both the amino group and the hydroxyl group of the 1,2-aminoalcohol; is adjacent to a multiple bond, such as a double or a triple bond, an aromatic ring or a heteroaromatic ring,
- the hydroxyl or the ether group, in particular both the ether group and the hydroxyl group of the 1,2-diol monoether, is adjacent to a multiple bond, such as a double or a triple bond, an aromatic ring or a heteroaromatic ring,
- the hydroxyl of the α-hydroxy-carbonyl compound is adjacent to a multiple bond, such as a double or a triple bond, an aromatic ring or a heteroaromatic ring,
- at least one of the carbonyl group is adjacent to a multiple bond, such as a double or a triple bond, an aromatic ring or a heteroaromatic ring,
- the oxygen atom of the epoxide is bound to at least one carbon atom, in particular to two carbon atoms adjacent to a multiple bond, such as a double or a triple bond, an aromatic ring or a heteroaromatic ring.

By "adjacent to a multiple bond, such as a double or a triple bond, an aromatic ring or a heteroaromatic ring", it is meant in the sense of the present invention that at least one of the hydroxyl, amino or carbonyl group is in a benzylic, propargylic or allylic position.

In a third aspect, the present invention concerns a process for preparing a carbonyl derivative, comprising the step of bringing into contact a 1,2-diol, a 1,2-diol monoether, a 1,2-diamine, a 1,2-aminoalcohol or an α-hydroxy-carbonyl compound in particular selected from an α-hydroxy-acid or a derivative thereof such as an α-hydroxy-ester, an α-hydroxy-ketone and an α-hydroxy-aldehyde, an α-dicarbonyl compound such as an α-ketoaldehyde, α-diketone or glyoxal; or an epoxide, preferably a 1,2-diol or an α-hydroxy-acid with a manganese-X mixed oxide as defined above, and an oxidant, preferably a 1,2-diol or an α-hydroxy-carbonyl compound.

Advantageously, the present invention concerns a process for preparing a carbonyl derivative as defined above, wherein:
- at least one of the hydroxyl groups, in particular the two hydroxyl groups of the diol, is adjacent to a multiple bond, such as a double or a triple bond, an aromatic ring or a heteroaromatic ring,
- at least one of the amino groups of the 1,2-diamine, in particular the two amino groups of the diamine, is adjacent to a multiple bond, such as a double or a triple bond, an aromatic ring or a heteroaromatic ring,
- the amino group or the hydroxyl group, in particular both the amino group and the hydroxyl group of the 1,2-aminoalcohol; is adjacent to a multiple bond, such as a double or a triple bond, an aromatic ring or a heteroaromatic ring,
- the hydroxyl or the ether group, in particular both the ether group and the hydroxyl group of the 1,2-diol monoether, is adjacent to a multiple bond, such as a double or a triple bond, an aromatic ring or a heteroaromatic ring,
- the hydroxyl of the α-hydroxy-carbonyl compound is adjacent to a multiple bond, such as a double or a triple bond, an aromatic ring or a heteroaromatic ring,
- at least one of the carbonyl group of the α-dicarbonyl compound is adjacent to a multiple bond, such as a double or a triple bond, an aromatic ring or a heteroaromatic ring,
- the oxygen atom of the epoxide is bound to at least one carbon atom, in particular to two carbon atoms adjacent to a multiple bond, such as a double or a triple bond, an aromatic ring or a heteroaromatic ring,
- an α-dicarbonyl compound wherein at least one of the carbonyl groups is adjacent to a multiple bond, such as a double or a triple bond, an aromatic ring or a heteroaromatic ring.

The present invention hence advantageously concerns a process as defined above, wherein at least one the oxygen atom or nitrogen atom of the starting 1,2-diol, 1,2-diol monoether, 1,2-diamine, 1,2-aminoalcohol, α-hydroxy-carbonyl compound, α-dicarbonyl compound or epoxide, preferably the 1,2-diol or the α-hydroxy-acid is in a benzylic, allylic or propargylic position with a manganese-X mixed oxide as defined above, and an oxidant, preferably a 1,2-diol or an α-hydroxy-carbonyl compound.

### Definitions:

Unless specifically indicated, throughout the specification and the appended claims, a given chemical formula or name shall encompass tautomers and all stereo, optical and geometrical isomers (e.g. enantiomers, diastereomers, E/Z isomers etc.) and racemates thereof as well as mixtures in different proportions of the separate enantiomers, mixtures of diastereomers,
or mixtures of any of the foregoing forms where such isomers and enantiomers exist.

The term "substituted" as used herein means that any one or more hydrogen(s) on the designated atom or group is replaced with a member of the indicated group of substituents, provided that the designated atom's normal valence is not exceeded. By the term "optionally substituted" is meant that either the corresponding group is substituted or it is not.

The term "alkyl" denotes an acyclic, saturated, branched or linear hydrocarbon radical. Examples of alkyl groups are methyl, ethyl, propyl, butyl, hexyl, heptyl and octyl. Said alkyl group may be optionally substituted, in particular with one or more (C₁-C₃)alkyl-halogen, such as difluoromethyl, trifluoromethyl, and pentafluoroethyl, hydroxyl, alkoxy, halogen, in particular fluor, amino, amido, nitro, cyano, carboxylic acid or a derivative thereof such as an ester or an amide or oxo. The term "alkyl" also comprises, within the sense of the present invention alkyl chains in which

The term "alkenyl" denotes an acyclic, straight or branched hydrocarbon radical wherein at least one single bond is replaced with a double bond. Examples of alkenyl groups are propenyl, butenyl or pentenyl. Said alkenyl group may be optionally substituted, in particular with one or more (C₁-C₃)alkyl, (C₁-C₃)alkyl-halogen, such as difluoromethyl, trifluoromethyl, and pentafluoroethyl, hydroxyl, alkoxy, halogen, in particular F, amino, amido, nitro, cyano, carboxylic acid or a derivative thereof such as an ester or an amide, or oxo.

The term "alkynyl" denotes an acyclic, straight or branched hydrocarbon radical wherein at least one single bond is replaced with a triple bond. Examples of alkynyl groups are propynyl, butynyl or pentynyl. Said alkynyl group may be optionally substituted, in particular with one or more (C₁-C₃)alkyl, (C₁-C₃)alkyl-halogen, such as difluoromethyl, trifluoromethyl, and pentafluoroethyl, hydroxyl, alkoxy, halogen, in particular F, amino, amido, nitro, cyano, carboxylic acid or a derivative thereof such as an ester or an amide, or oxo.

The term "cycloalkyl" denotes a cyclic, saturated, unbranched hydrocarbon radical. Examples of cycloalkyl groups are cyclopropyl, cyclobutyl, cyclopentyl, cyclohexyl, cycloheptyl and cyclooctyl. Said cycloalkyl group may be optionally substituted, in particular with one or more (C₁-C₃)alkyl, (C₁-C₃)alkyl-halogen, such as difluoromethyl, trifluoromethyl, and pentafluoroethyl, hydroxyl, alkoxy, halogen, in particular F, amino, amido, nitro, cyano, carboxylic acid or a derivative thereof such as an ester or an amide, or oxo.

The term "aryl" as used herein denotes a carbocyclic aromatic monocyclic group containing 6 carbon atoms which may be further fused to a second 5- or 6-membered carbocyclic group which may be aromatic, saturated or unsaturated. Within the meaning of the present invention, the term aryl also includes biaryl such as biphenyl. Examples of aryl groups are phenyl, indanyl, indenyl, naphthyl, anthracenyl, phenanthrenyl, tetrahydronaphthyl and dihydronaphthyl. Said aryl group may be optionally substituted, in particular with one or more (C₁-C₃)alkyl, (C₁-C₃)alkyl-haiogen, such as difluoromethyl, trifluoromethyl, and pentafluoroethyl, hydroxyl, alkoxy, halogen, in particular F, amino, amido, nitro, cyano, carboxylic acid or a derivative thereof such as an ester or an amide.

The term "heteroaryl" means an aromatic 5 to 6-membered monocyclic heteroaryl or an aromatic 7 to 11-membered heteroaryl bicyclic ring where at least one of the rings is aromatic, wherein the heteroaryl ring contains 1-4 heteroatoms such as N, O and S. examples of 5 to 6-membered monocyclic heteroaryl rings are furanyl, oxazolyl, isoxazolyl, oxadiazolyl, thiazolyl, pyrazolyl, pyrrolyl, imidazolyl, tetrazolyl, triazolyl, thienyl, thiadiazolyl, pyridinyl, pyrimidinyl, pyridazinyl, pyrazinyl, triazinyl, and purinyl. Examples of 7 to 11-membered heteroaryl bicyclic heteroaryl rings are benzimidazolyl, quinolinyl, dihydro-2H-quinolinyl, tetrahydroquinolinyl, isoquinolinyl, quinazolinyl, indazolyl, thieno[2,3- d]pyrimidinyl, indolyl, isoindolyl, benzofuranyl, dihydrobenzofuranyl, benzopyranyl, benzodioxolyl, benzoxazolyl and benzothiazolyl. Said heteroaryl group may be optionally substituted, in particular with one or more (C₁-C₃)alkyl, (C₁-C₃)alkyl-halogen, such as difluoromethyl, trifluoromethyl, and pentafluoroethyl, hydroxyl, alkoxy, halogen, in particular F, amino, amido, nitro, cyano, carboxylic acid or a derivative thereof such as an ester or an amide.

The term "heterocycloalkyl" means a stable nonaromatic 4-8 membered monocyclic heterocyclic radical or a stable nonaromatic 6 to 11-membered fused bicyclic, bridged bicyclic or spirocyclic heterocyclic radical. The 5 to 11-membered heterocycle consists of carbon atoms and one or more, preferably from one to four heteroatoms chosen from nitrogen, oxygen and sulfur. The heterocycle may be either saturated or partially unsaturated. Non-limiting examples of nonaromatic 4-8 membered monocyclic heterocyclic radicals include tetrahydrofuranyl, azetidinyl, pyrrolidinyl, pyranyl, tetrahydropyranyl, dioxanyl, thiomorpholinyl, I,I-dioxo-1<6>- thiomorpholinyl, morpholinyl, piperidinyl, piperazinyl, and azepinyl. Examples of nonaromatic 6 to 11-membered fused bicyclic radicals include octahydroindolyl, octahydrobenzofuranyl, and octahydrobenzothiophenyl. Examples of nonaromatic 6 to 11-membered bridged bicyclic radicals include 2-azabicyclo[2.2.1]heptanyl, 3- azabicyclo[3.1.0]hexanyl, and 3-azabicyclo[3.2.1]octanyl. Non-limiting examples of nonaromatic 6 to 11-membered spirocyclic heterocyclic radicals include 7-azaspiro[3,3]heptanyl, 7-spiro[3,4]octanyl, and 7-aza-spiro[3,4]octanyl. The term "heterocycloalkyl" is intended to include all the possible isomeric forms. Said alkenyl group may be optionally substituted, in particular with one or more (C₁-C₃)alkyl, (C₁-C₃)alkyl-halogen, such as difluoromethyl, trifluoromethyl, and pentafluoroethyl, hydroxyl, alkoxy, halogen, in particular F, amino, amido, nitro, cyano, carboxylic acid or a derivative thereof such as an ester or an amide, or oxo.

The terms "alkyl-aryl", "alkenyl-aryl", "alkynyl-aryl", "alkyl-heteroaryl", "alkenyl-heteroaryl" and "alkynyl-heteroaryl" denote two groups as defined above bound together.

Particular examples of such groups are the following: 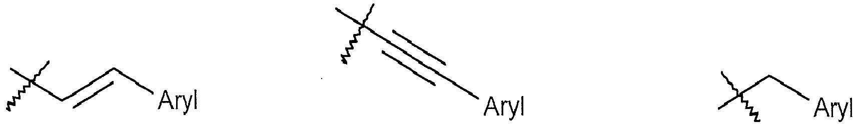 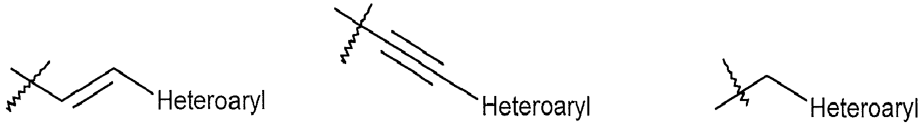 wherein the aryl is in particular a phenyl group optionally substituted in particular with one or more hydroxyl groups.

In one embodiment, the present invention concerns a process for preparing a carbonyl derivative, wherein the 1,2-diol, 1,2-diol monoether, 1,2-diamine or 1,2-aminoalcohol has formula (I): 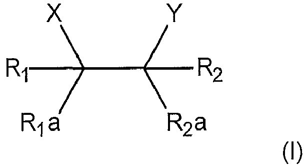 wherein:
X and Y, identical or different, are selected from OH, OR₃ and NHR₄,
R₁, R₂, R₁ₐ and R₂ₐ, identical or different, are selected from the group consisting of H, alkyl, alkenyl, alkynyl, aryl, heteroaryl, cycloalkyl, heterocycloalkyl, alkyl-aryl, alkenyl-aryl, alkynyl-aryl, alkyl-heteroaryl, alkenyl-heteroaryl and alkynyl-heteroaryl, at least one of said groups being different from H,
R₃ is selected from the group consisting of alkyl, cycloalkyl, heterocycloalkyl, alkyl-aryl and alkyl-heteroaryl,
R₄ is selected from the group consisting of H, alkyl, aryl, heteroaryl, cycloalkyl, heterocycloalkyl, alkyl-aryl, alkenyl-aryl, alkynyl-aryl, alkyl-heteroaryl, alkenyl-heteroaryl and alkynyl-heteroaryl,
to give a first carbonyl derivative of formula (II): 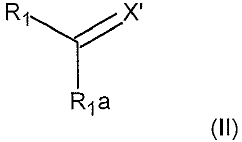 and a second carbonyl derivative of formula (III): 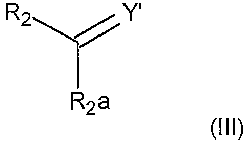
wherein R₁, R₂, R₁ₐ et R₂ₐ are as defined in formula (I), and
X' and Y', identical or different, represent O or NR₄.

In a particular embodiment, the compound of formula (I) has formula (Ia): 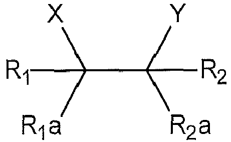 wherein:
R₂ and R₂ₐ are as defined in formula (I),
R₁ is H,
R₁ₐ is selected from an aryl, an heteroaryl and the following groups: 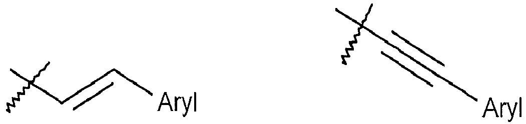 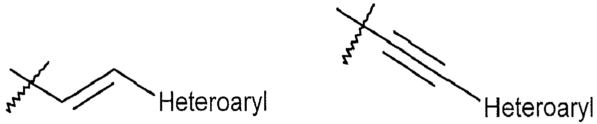
wherein the aryl is in particular an optionally substituted phenyl group. Advantageously, said compound of formula (I) is a 1,2-diol and X and Y are OH. In another particular embodiment, the compound of formula (I) has formula (Ib): 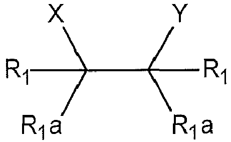 wherein:
X and Y are as defined in formula (I),
R₁ and R₁ₐ identical or different, are selected from H, aryl, heteroaryl, and the following groups: 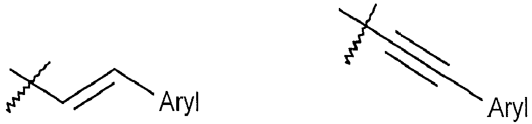 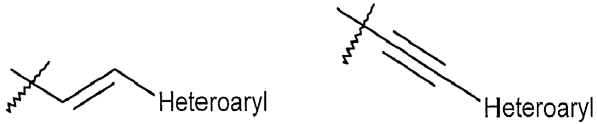 wherein the aryl is in particular a phenyl group optionally substituted, in particular with one or more hydroxyl groups.
with the proviso that at least one R₁ group or one R₁ₐ group is different from H.

Advantageously, the compound of formula (Ib) is selected from the following compounds: 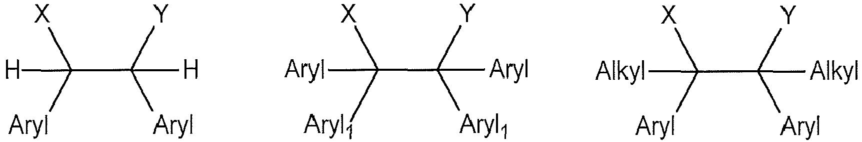 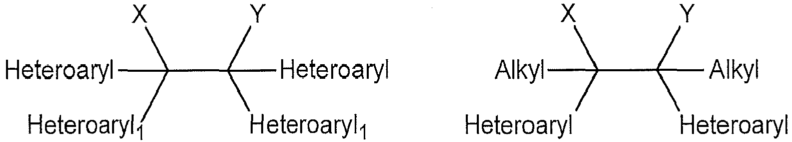 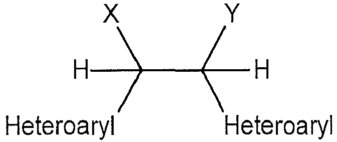 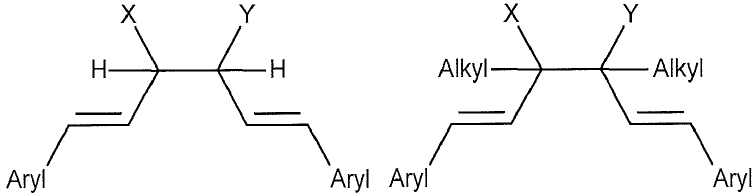 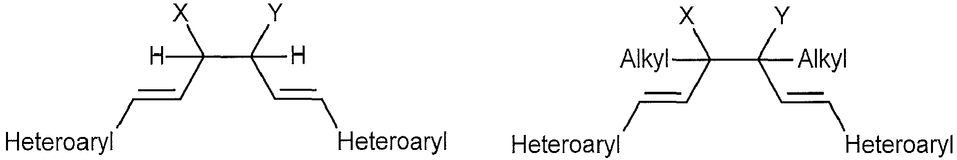 wherein the groups heteroaryl and heteroaryl₁ are identical or different and the groups aryl and aryl₁ are identical or different. Preferably, the groups heteroaryl and heteroaryl₁ are identical and the groups aryl and aryl₁ are identical.

More preferably, the compound of formula (Ib) is selected from the group consisting of: 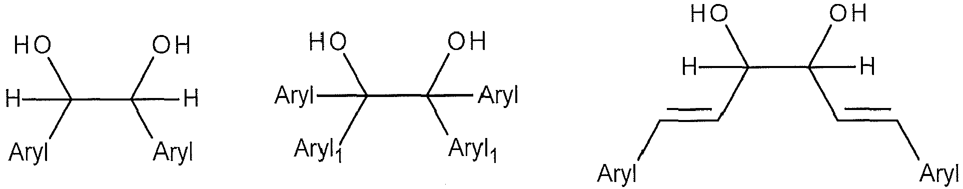 wherein the aryl is in particular a phenyl.

In another embodiment, the present invention concerns a process for preparing a carbonyl derivative, wherein the epoxide has formula (IV): 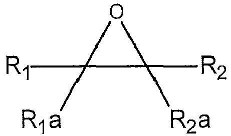 wherein:
R₁, R₂, R₁ₐ and R₂ₐ, identical or different, are selected from the group consisting of H, alkyl, alkenyl, alkynyl, aryl, heteroaryl, cycloalkyl, heterocycloalkyl, alkyl-aryl, alkenyl-aryl, alkynyl-aryl, alkyl-heteroaryl, alkenyl-heteroaryl and alkynyl-heteroaryl.
to give a first carbonyl derivative of formula (II): 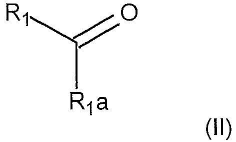 and a second carbonyl derivative of formula (III): 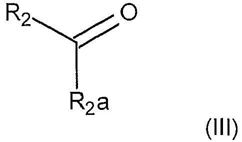 wherein R₁, R₂, R₁ₐ et R₂ₐ are as defined in formula (I).

In a particular embodiment, the epoxide has formula (IVa): 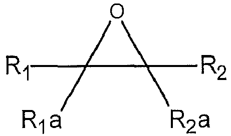 wherein one of R₁ and R₁ₐ is selected from the group consisting of aryl, heteroaryl and a group selected from:

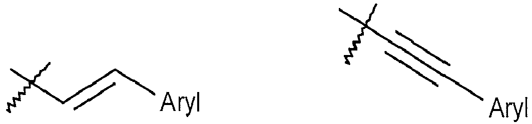

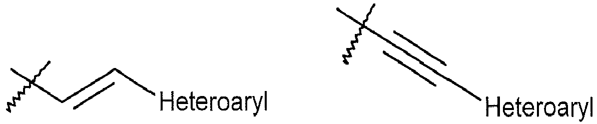

In yet another embodiment, the present invention concerns a process for preparing a carbonyl derivative, wherein the α-hydroxy-carbonyl compound has formula (V): 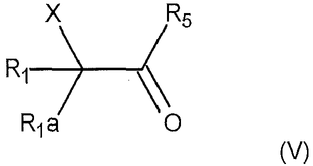 wherein:
X is selected from OH and NHR₄,
R₁ and R₁ₐ, identical or different, are selected from the group consisting of H, alkyl, alkenyl, alkynyl, aryl, heteroaryl, cycloalkyl, heterocycloalkyl, alkyl-aryl, alkenyl-aryl, alkynyl-aryl, alkyl-heteroaryl, alkenyl-heteroaryl and alkynyl-heteroaryl,
R₅ is selected from the group consisting of H, OH, OR_{b}, NR_{c}R_{d}, alkyl, alkenyl, alkynyl, aryl, heteroaryl, cycloalkyl, heterocycloalkyl, alkyl-aryl, alkenyl-aryl, alkynyl-aryl, alkyl-heteroaryl, alkenyl-heteroaryl and alkynyl-heteroaryl, in which R_{b}, R_{c} and R_{d} are selected from the group consisting of alkyl, alkenyl, alkynyl, aryl, heteroaryl, cycloalkyl, heterocycloalkyl, alkyl-aryl, alkenyl-aryl, alkynyl-aryl, alkyl-heteroaryl, alkenyl-heteroaryl and alkynyl-heteroaryl or R_{c} and R_{d} form together with the nitrogen atom to which they are linked a heterocycloalkyl,
to give a carbonyl derivative of formula (II): 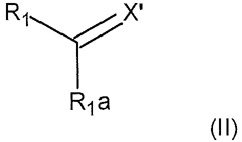
wherein R₁ and R₁ₐ are as defined in formula (V), and
X' represents O or NR₄.

In one particular embodiment, the α-hydroxy-carbonyl compound has formula (Va): 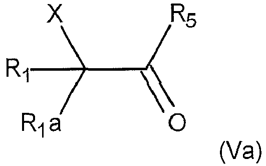 wherein X is OH and one of R₁ and R₁ₐ is selected from the group consisting of aryl, heteroaryl, alkenyl-aryl and alkenyl-heteroaryl, in particular from the group consisting of aryl, heteroaryl and the following compounds:

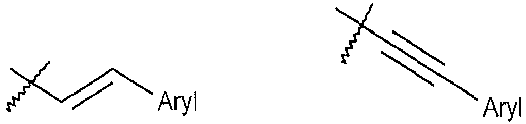

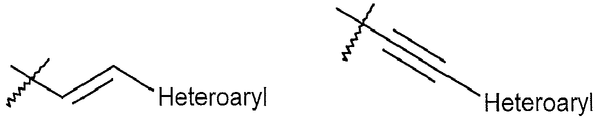

Said α-hydroxy-carbonyl may in particular be an α-hydroxy-acid of the following formula (Va1):

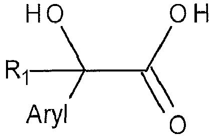

An example of such an hydroxy-acid is mandelic acid and derivatives thereof.

In yet a further embodiment, the present invention concerns a process for preparing a carbonyl derivative, wherein the α-dicarbonyl compound is an α-ketoaldehyde, an α-diketone, glyoxal or an an α-keto-acid or a derivative thereof having formula (VI): 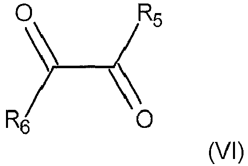 wherein:
R₅ is selected from the group consisting of H, OH, OR_{b}, NR_{c}R_{d}, alkyl, alkenyl, alkynyl, aryl, heteroaryl, cycloalkyl, heterocycloalkyl, alkyl-aryl, alkenyl-aryl, alkynyl-aryl, alkyl-heteroaryl, alkenyl-heteroaryl and alkynyl-heteroaryl, in which R_{b}, R_{c} and R_{d} are selected from the group consisting of alkyl, alkenyl, alkynyl, aryl, heteroaryl, cycloalkyl, heterocycloalkyl, alkyl-aryl, alkenyl-aryl, alkynyl-aryl, alkyl-heteroaryl, alkenyl-heteroaryl and alkynyl-heteroaryl or R_{c} and R_{d} form together with the nitrogen atom to which they are linked a heterocycloalkyl,
R₆ is selected from the group consisting of H, alkyl, alkenyl, alkynyl, aryl, heteroaryl, cycloalkyl, heterocycloalkyl, alkyl-aryl, alkenyl-aryl, alkynyl-aryl, alkyl-heteroaryl, alkenyl-heteroaryl and alkynyl-heteroaryl,
to give a carboxylic acid of formula (VII): 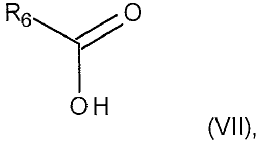 wherein R₆ is as defined in formula (VI).

Advantageously, the α-dicarbonyl compound is an α-ketoaldehyde or an α-diketone of formula (Via) wherein R₄ and R₅, identical or different, are selected from the group consisting of H, alkyl, alkenyl, alkynyl, aryl, heteroaryl, cycloalkyl, heterocycloalkyl, alkyl-aryl, alkenyl-aryl, alkynyl-aryl, alkyl-heteroaryl, alkenyl-heteroaryl and alkynyl-heteroaryl. More advantageously, the compound is an α-ketoaldehyde wherein R₅ is H.

### Conditions for performing the process

In the process defined above, the amount of the manganese-X mixed oxide used as catalyst may be determined by the person skilled in the art depending on the substrate, the reaction rate and other requirements. Said amount typically ranges from 1 part per million (ppm) to 1 equivalent, advantageously 50 ppm to 0.5 equivalent, preferably 100 ppm to 0.1 equivalent with respect to the 1,2-diol, 1,2-diamine, 1,2-aminoalcohol, α-hydroxy-carbonyl compound, α-dicarbonyl compound or epoxide.

In one embodiment, the present invention concerns a process as defined above, characterized in that the oxidative cleavage is performed in the presence of a base.

It has been evidenced by the Inventors that when none or only one of R₁, R₂, R₁ₐ and R₂ₐ is one of the following groups: 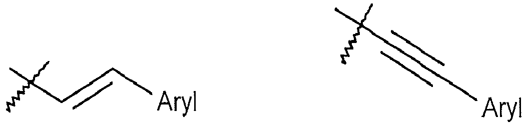 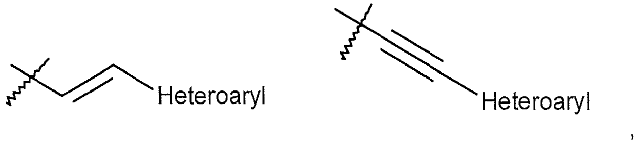 i.e. when the hydroxyl, amino or carbonyl is not in allylic, propargylic or benzylic position, the yield of the carbonyl product may be improved by the addition of a base to the reaction medium.

Said base may in particular be selected from the group consisting of carbonates, such as sodium carbonate, potassium carbonate or lithium carbonate or a hydroxide, such as sodium hydroxide, lithium hydroxide or potassium hydroxide.

When the base is an hydroxide, it is preferable to add it to the reaction mixture in substoechiometric amount with respect to the 1,2-diol, the 1,2-diamine, the 1,2-aminoalcohol, the α-hydroxy-carbonyl compound, the α-dicarbonyl compound or the epoxide.

When the base is a carbonate, it is preferably used in amounts of 0.1 to 10 equivalents, advantageously 0.1 to 5 equivalents, preferably of 2 equivalents, with respect to the 1,2-diol, the 1,2-diamine, the 1,2-aminoalcohol, the α-hydroxy-carbonyl compound, the α-dicarbonyl compound or the epoxide (molar ratio).

When the base is a hydroxide, it is preferably used in amounts of 0.1 to 1 equivalent, advantageously 0.1 to 0.5 equivalents, preferably of 0.1 equivalent, with respect to the 1,2-diol, the 1,2-diamine, the 1,2-aminoalcohol, the α-hydroxy-carbonyl compound, the α-dicarbonyl compound or the epoxide (molar ratio).

The oxidative cleavage can be performed with any suitable oxidant. Preferably, said oxidant is pure dioxygen or air.

The reaction may be performed in any suitable solvent. Preferably, it is performed in a polar solvent such as an alcohol, N,N-dimethylformamide, acetonitrile or ethyl acetate.

Advantageously, the solvent is an alcohol such as methanol, ethanol, propanol or butanol.

The reaction may be performed at any suitable temperature. It is typically performed at a temperature ranging from 10 to 160 °C, advantageously from 50 to 120 °C, preferably from 90 °C to 110 °C.

In a third aspect, the present invention concerns a process for preparing a Manganese-X mixed oxide, wherein the manganese is at the oxidation state +III and +IV and X is a cation selected from the group consisting of sodium, calcium, magnesium, cesium, lithium, potassium, iron, zinc, strontium, cobalt, cerium, vanadium, molybdenum, tin, tungsten, titanium, chromium, nickel, copper, ruthenium, bismuth, antimony, aluminum, silver, rhenium, osmium, rhodium, iridium, palladium, gold and tetramethylammonium, characterized in that:
(a) an hydroxide of formula Xⁿ⁺(HO⁻)ₙ is added to a solution containing a Manganese (II) salt and an oxidant, or
(b) an hydroxide of formula M^{m+}(HO⁻)ₘ is added to a solution containing a manganese (II) salt, an oxidant and a compound of formula Xⁿ⁺(Y^{p-})_{n/p} in which M is a metal selected in particular from Li, K and Na and Y is a neutral counterion such as a chloride or a sulfate and n, m and p are the charge of the corresponding ion.

The process according to the present invention differs from the ones disclosed in prior art essentially in that an hydroxide is added to a solution containing a manganese salt and an oxidant instead of adding the oxidant to a solution containing a Manganese (II) salt and an hydroxide.

It has unexpectedly been found by the Inventor that selection of this order of addition of the reactants results in the formation of a different material, as evidenced by the improved activity of the material in the oxidative cleavage of 1,2-diols as compared to the material obtained when the base is added before the oxidant. Alternatively, one may add a mixture of the hydroxide and the oxidant to the Manganese (II) salt and, when present, the compound of formula Xⁿ⁺(Y^{p-})_{n/p}. It is preferred that the base is added after the oxidant.

In an advantageous embodiment, said cation X is selected from the group consisting of sodium, calcium, magnesium, cesium, lithium, potassium, iron, tetramethylammonium, strontium and zinc.

In a more advantageous embodiment, the cation X is selected from the group consisting of sodium, calcium, strontium and zinc.

In the process according to the present invention, the Manganese (II) salt is advantageously selected from the croup consisting of the chloride, the nitrate, the sulfate, the acetate, the carbonate, the bromide, the iodide, the phosphate, the oxalate, the sulfide, the acetyl acetonate, the formate and the gluconate. Preferably, the manganese (II) salt is the chloride.

The oxidant may be any suitable oxidant. Advantageously, the oxidant is selected from the group consisting of hydrogen peroxide, a peracid, an alkyl peroxide, dioxygen, air and a persulfate. Preferably, the oxidant is hydrogen peroxide, dioxygen or air, most preferably hydrogen peroxide.

The oxidant is typically present in proportion of 2:1 to 10:1, advantageously 3:1 to 5:1, preferably 3:1, molar with respect to the manganese (II) salt. When the oxidant is a gas, such as air or dioxygen, the process may be performed at atmospheric pressure or at a pressure superior to atmospheric pressure.

In a first embodiment, the process for preparing the Manganese-X mixed oxide, wherein the manganese is at the oxidation state +III and +IV and X is a cation selected from the group consisting of sodium, calcium, magnesium, cesium, lithium, potassium, iron, zinc, strontium, cobalt, cerium, vanadium, molybdenum, tin, tungsten, titanium, chromium, nickel, copper, ruthenium, bismuth, antimony, aluminum, silver, rhenium, osmium, rhodium, iridium, palladium, gold and tetramethylammonium comprises the step of adding an hydroxide of formula Xⁿ⁺(HO⁻)ₙ to a solution containing a Manganese (II) salt and an oxidant.

As a non-limiting example of this first embodiment, the Manganese-Na mixed oxide is prepared by adding sodium hydroxide to the solution containing the Manganese (II) salt and the oxidant.

In a second embodiment, the process for preparing the Manganese-X mixed oxide, wherein the manganese is at the oxidation state +III and +IV and X is a cation selected from the group consisting of sodium, calcium, magnesium, cesium, lithium, potassium, iron, zinc, strontium, cobalt, cerium, vanadium, molybdenum, tin, tungsten, titanium, chromium, nickel, copper, ruthenium, bismuth, antimony, aluminum, silver, rhenium, osmium, rhodium, iridium, palladium, gold and tetramethylammonium comprises the step of adding an hydroxide of formula M^{m+}(HO⁻)ₘ to a solution containing the manganese (II) salt, the oxidant and further containing a salt of formula Xⁿ⁺(Y^{p-})_{n/p} in which X is as defined above, M is a metal selected in particular from Li, K and Na and Y is a neutral counterion such as a chloride or a sulfate.

In the sense of the present invention, a "neutral counterion" is defined as an ion that does not cause a variation of the pH of the solution, in particular that does not cause a pH increase (*i.e.* towards a pH of 14). It is indeed speculated that upon addition of a base, the Manganese (II) salt is immediately oxidized into Manganese (III) and Manganese (IV) in the presence of the oxidant.

According to this embodiment, the cation X is mixed with the manganese (II) salt and the oxidant and a hydroxide in which M is different from X is added. This procedure may in particular be useful when the corresponding hydroxide of the cation X is unstable, insoluble or cannot be prepared in a straightforward way.

As a non-limiting example of this second embodiment, a Manganese-Zn mixed oxide is prepared by adding sodium hydroxide to the solution containing the Manganese (II) salt, the oxidant and zinc chloride.

In the process for preparing the Manganese-X mixed oxide, wherein the manganese is at the oxidation state +III and +IV and X is a cation selected from the group consisting of sodium, calcium, magnesium, cesium, lithium, potassium, iron, zinc, strontium, cobalt, cerium, vanadium, molybdenum, tin, tungsten, titanium, chromium, nickel, copper, ruthenium, bismuth, antimony, aluminum, silver, rhenium, osmium, rhodium, iridium, palladium, gold and tetramethylammonium, the amount of hydroxide added vary from 2:1 to 80:1, advantageously 4:1 to 60:1, preferably 20:1 to 50:1, molar with respect to the manganese (II) salt. Preferably, the hydroxide is added at a proportion of 42:1, molar with respect to the manganese (II) salt.

In a more particular embodiment, the present invention provides a process for preparing a Manganese-Na mixed oxide, wherein the manganese is at the oxidation state +III and +IV, characterized in that sodium hydroxide is added to a solution of a manganese (II) salt as defined above and an oxidant.

Advantageously, sodium hydroxide is added in proportion of 4:1 to 50:1, in particular 42:1, molar with respect to the manganese (II) salt.

Advantageously, the manganese (II) salt is the chloride salt.

Advantageously, the oxidant is pure dioxygen, hydrogen peroxide or air, preferably hydrogen peroxide. Preferably, hydrogen peroxide is used in proportion of 3:1, molar with respect to the manganese (II) salt.

The present invention also concerns a Manganese-X mixed oxide obtainable by the process described above.

In a fourth aspect, the present invention concerns a Manganese-X mixed oxide, wherein the manganese is at the oxidation state +III and +IV and X is sodium, characterized in that manganese is present in proportion of 45 to 55 % and sodium is present in proportion of 1 to 10 %, advantageously 5 to 7%, by weight of the total weight of said mixed oxide. The manganese in said Manganese-X mixed oxide is composed of between about 20 and about 40 mol% of Mn(III) and between 60 and 80 mol% of Mn(IV). In particular, the Manganese-X mixed oxide is composed of about 30 mol% of Mn(III) and about 70 mol% of Mn(IV), based on the total Manganese present, as determined by mineral analysis and thermogravimetric analysis as described in the examples. Said manganese hence has a charge of + 3,7 (according to J. AM. CHEM. SOC. 2008, 130, 15938-15943).

Said Manganese-Na mixed oxide is essentially devoid of Manganese (II). By "essentially devoid of Manganese (II)", it is meant in the sense of the present invention that the spectrum of the Manganese-X mixed oxide is devoid of the specific Mn2p peaks at a value lower than 641 eV and a satellite peak at about 647 eV (Geochimica et Cosmochimica Acta Vol. 58, No. 22, pp. 4985-4999. 1994) when analyzed by X-Ray photoelectron spectrometry (XPS).

The present invention concerns a Manganese-X mixed oxide, wherein the manganese is at the oxidation state +III and +IV and X is sodium, characterized in that manganese is present in proportion of 45 to 55 % and sodium is present in proportion of 1 to 10 %, advantageously 5 to 7%, by weight of the total weight of said mixed oxide may be in particular obtained by the process according to the present invention, as defined above. The manganese in said Manganese-X mixed oxide is composed of between about 20 and about 40 mol% of Mn(III) and between 60 and 80 mol% of Mn(IV). In particular, the Manganese-X mixed oxide is composed of about 30 mol% of Mn(III) and about 70 mol% of Mn(IV), based on the total Manganese present, as determined by mineral analysis and thermogravimetric analysis as described in the examples. Said manganese hence has a charge of + 3,7 (according to J. AM. CHEM. SOC. 2008, 130, 15938-15943).

The present invention therefore concerns a Manganese-X mixed oxide, wherein the manganese is at the oxidation state +III and +IV and X is sodium, characterized in that manganese is present in proportion of 45 to 55 % and sodium is present in proportion of 1 to 10 %, advantageously 5 to 7%, by weight of the total weight of said mixed oxide obtained by a process comprising the step of adding NaOH to a solution containing a Manganese (II) salt and an oxidant. Advantageously, sodium hydroxide is added in proportion of 4:1 to 50:1, in particular 42:1, molar with respect to the manganese (II) salt. Advantageously, the manganese (II) salt is the chloride salt. Advantageously, the oxidant is pure dioxygen, hydrogen peroxide or air, preferably hydrogen peroxide. Preferably, the oxidant is used in proportion of 3:1, molar with respect to the manganese (II) salt. More preferably, the Manganese-X mixed oxide is devoid of Mn at the oxidation state +II. The manganese in said Manganese-X mixed oxide is composed of between about 20 and about 40 mol% of Mn(III) and between 60 and 80 mol% of Mn(IV). In particular, the Manganese-X mixed oxide is composed of about 30 mol% of Mn(III) and about 70 mol% of Mn(IV), based on the total Manganese present, as determined by mineral analysis and thermogravimetric analysis as described in the examples. Said manganese hence has a charge of + 3,7 (according to J. AM. CHEM. SOC. 2008, 130, 15938-15943).

When employing said process, the Manganese-Na mixed oxide defined above is in the form of particles having a diameter of 1 nm to 100 µm, notably 150 to 500 nm, in particular 50 to 150 nm. The process also enables obtaining a material having a layered structure.

Said layered structure may be characterized by analytical methods known in the art, in particular by X-ray diffraction (by indexing the spectra with other known layered manganese oxides) or infrared spectroscopy as already described in Spectrochimica Acta Part A 67 (2007) 864-869*.*

In one embodiment, the Manganese-Na mixed oxide as defined above is a layered material in amorphous state.

In a further embodiment, the present invention concerns the use of the Manganese-Na mixed oxide as defined above, as an oxidation catalyst for implementing oxidation reactions.

Said oxidation reaction may in particular be selected from the group consisting of the oxidative cleavage of a 1,2-diol, a 1,2-diamine, a 1,2-aminoalcohol or an α-hydroxy-carbonyl compound, in particular selected from an α-hydroxy-acid or a derivative thereof such as an α-hydroxy-ester, an α-hydroxy-ketone and an α-hydroxy-aldehyde, an α-dicarbonyl compound such as an α-ketoaldehyde, α-diketone or glyoxal; or an epoxide, preferably a 1,2-diol or an α-hydroxy-acid; the oxidation of sulfides into sulfoxides or sulfones; the formation of azo compounds from anilines; the C-H oxidation of aromatic rings; and the oxidation of alcohols, in particular of allylic, benzylic and propargylic alcohols.

In a particular embodiment, the present invention concerns the use of a Manganese-Na mixed oxide as defined above for the oxidative cleavage of a 1,2-diol, a 1,2-diamine, a 1,2-aminoalcohol or an α-hydroxy-carbonyl compound, in particular selected from an α-hydroxy-acid or a derivative thereof such as an α-hydroxy-ester, an α-hydroxy-ketone and an α-hydroxy-aldehyde, an α-dicarbonyl compound such as an α-ketoaldehyde, α-diketone or glyoxal, or an epoxide, preferably a 1,2-diol or an α-hydroxy-acid.

### Examples:

### Example I: Preparation of a Mn-Na mixed oxide (direct addition of an hydroxide):

In a 100 mL flask equipped with a magnetic stirring bar, 178.1 mg of MnCl₂.4H₂O (0.9 mmol) are put in solution in 9 mL of water.

Under stirring (400 rpm), 0.279 mL of hydrogen peroxide 30 % in water (2.7 mmol) is added at once. A colorless solution is obtained. Stirring is continued for a further 5 minutes and an aqueous solution of NaOH (1536 mg in 2 mL of water, 38.4 mmol) is added at once. A black precipitate forms immediately. The sides of the flask are rinsed with 10 mL of water and stirring is continued for 90 minutes at 800 rpm. The resulting suspension is filtered under vacuum on fiber glass (Whatman 4.25 cm diameter, no 1820042) and rinsed three times with 100 mL of water and once with 50 mL of ethanol. The precipitate should not be fully left to dry to preserve its activity. After rinsing, the precipitate is collected in air then in an oven at 105 °C for 18h. The obtained precipitate is finely divided with a mortar and stocked in a desiccator under vacuum.

### Example II: Preparation of a Mn-X mixed oxide (addition of an hydroxide to a solution containing a salt of X):

In a 100 mL flask equipped with a magnetic stirring bar, 178.1 mg of MnCl₂.4H₂O (0.9 mmol) and the mass of salt of X (according to table I) corresponding to 1.93 mmol of X are put in solution in 9 mL of water. Under stirring (400 rpm), 0.279 mL of hydrogen peroxide 30 % in water (2.7 mmol) is added at once. A colorless solution is obtained. Stirring is continued for a further 5 minutes and an aqueous solution of NaOH (1536 mg in 2 mL of water, 38.4 mmol) is added at once. A black precipitate forms immediately. The sides of the flask are rinsed with 10 mL of water and stirring is continued for 90 minutes at 800 rpm. The resulting suspension is filtered under vacuum on fiber glass (Whatman 4.25 cm diameter, no 1820042) and rinsed three times with 100 mL of water and once with 50 mL of ethanol. The precipitate should not be fully left to dry to preserve its activity. After rinsing, the precipitate is collected in air then in an oven at 105 °C for 18h. The obtained precipitate is finely divided with a mortar and stocked in a desiccator under vacuum.

**Table I:**

| **X** | **Salt** | **Amount of salt** |
|---|---|---|
| Zn | ZnCl₂ | 263 mg |
| Fe | FeCl₃.6H₂O | 522 mg |
| Sr | SrCl₂.6H₂O | 515 mg |
| Mg | MgCl₂.6H₂O | 392 mg |
| Ca | CaCl₂.2H₂O | 284 mg |

### Example 2: Analytical data of the Mn-Na mixed oxide according to example 1:

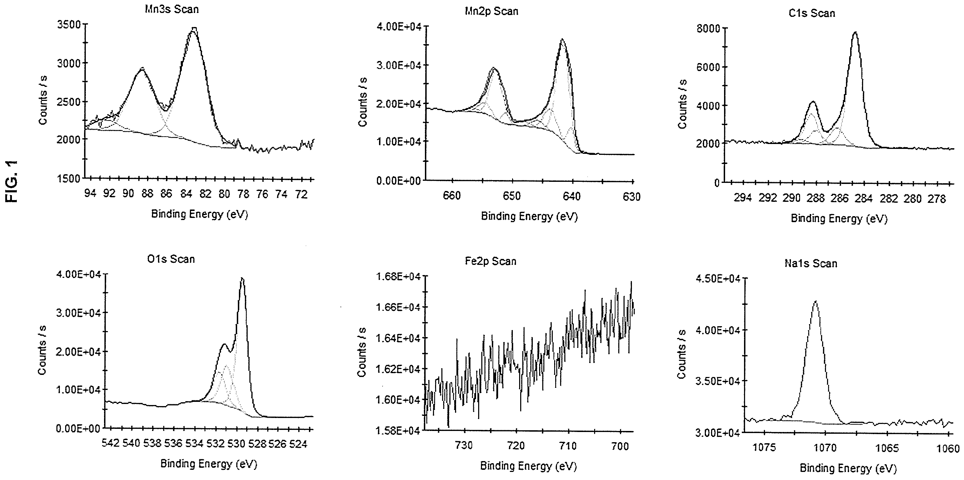

The precipitate has been analyzed by X-Ray photoelectron spectrometry (XPS). The corresponding spectra are shown in Figure 1. The data show that the precipitate contains Manganese (III) and Manganese (IV). No Manganese (II) is detected using this method.

The composition of the precipitate was analyzed by mineral analysis. The results are shown in table 2:

**Table 2**

| Element | Mn | Na |
|---|---|---|
| wt% in the sample | 52,7559 | 6,0472 |
| sd (wt%) | 0,6457 | 0,1638 |

The missing weight percentage corresponds to oxygen. A minor amount of water adsorbed at the surface is also present.

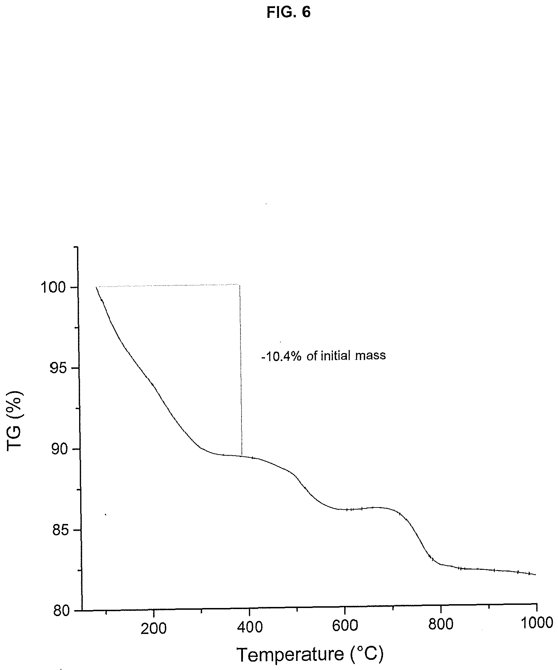

From these values, the composition of the precipitate may be determined as MnO_{2.68}Na_{0.274}. Thermogravimetric analysis was also employed to determine the amount of water included within the pores of the catalyst and thereby to determine the exact content in oxygen bound to manganese. The results are depicted in Figure 6. The composition of the catalyst determined using thermogravimetric analysis is Na_{0.274}MnO₂·0.6 H₂O.

Employing the method disclosed in J. AM. CHEM. SOC. 2008, 130, 15938-15943, the average degree of oxidation is +3.7. Said catalyst hence contains 30 % Mn(III) and 70 % Mn(IV) (molar ratio).

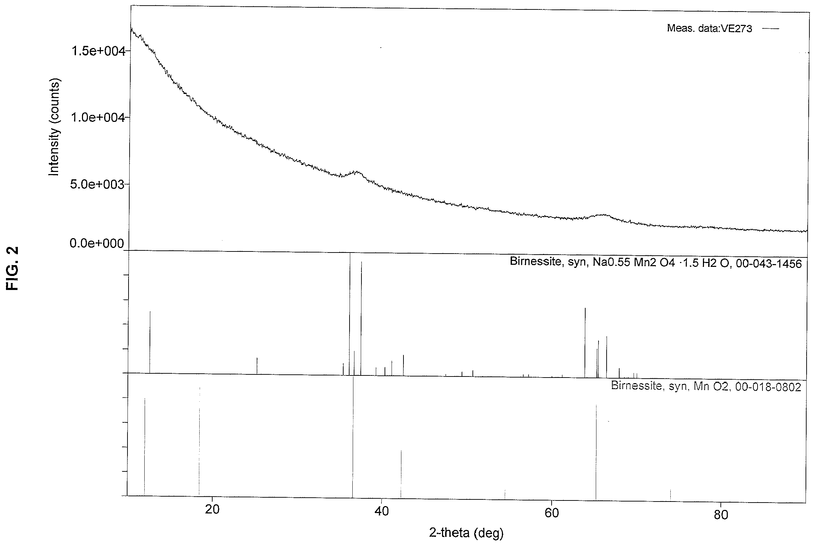

The precipitate has been analyzed by X-ray powder diffraction. The corresponding spectrum is shown in Figure 2 (upper curve), in comparison with the spectrum of birnessites (bottom and middle curves). Comparison with the spectra of known birnessites confirms that the obtained precipitate has a layered structure.

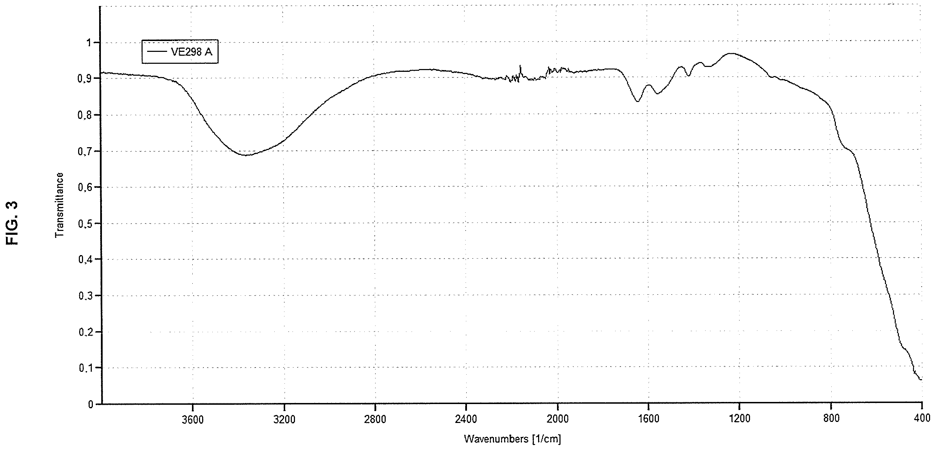

Infra-Red spectroscopy has also been used for the purpose of characterizing the precipitate. The corresponding spectrum is shown in Figure 3. The IR spectrum is characterized by an intense absorption at around 400 cm⁻¹ and the absence of absorption between 400 et 800 cm⁻¹. The absence of an absorption between 400 and 800, in particular at 761 (as explained in Spectrochimica Acta Part A 67 (2007) 864-869) indicates that the material has a layered structure.

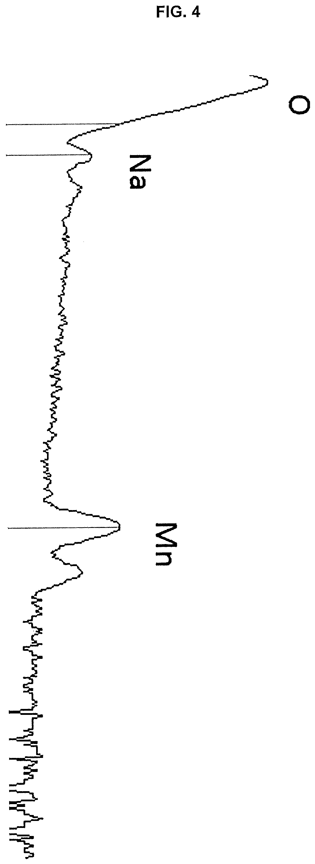

The precipitate has been further characterized by Energy Dispersive X-Ray Analysis (EDX). EDX (also referred to as EDS or EDAX) is an x-ray technique used to identify the elemental composition of materials. The corresponding spectrum is shown in Figure 4.

As shown by this spectrum, the precipitate contains Na and Mn.

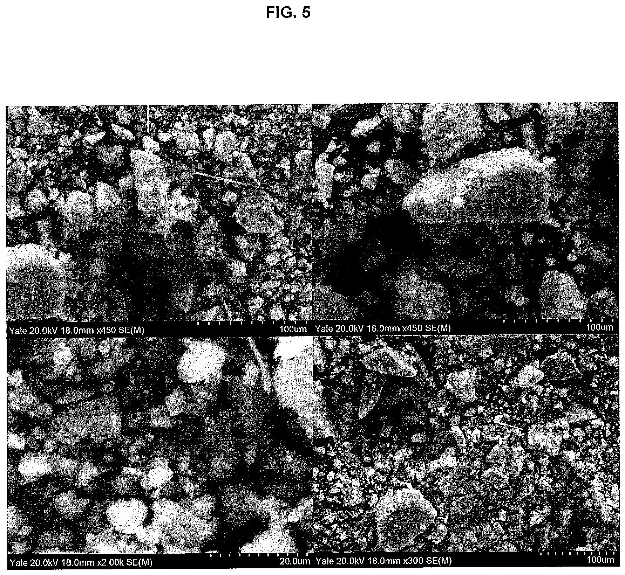

Figure 5 shows the SEM images of the precipitate. As can be seen, the precipitate contains aggregates having sizes of up to about 100 µm.

The size of the particles of the precipitate was further analyzed by dynamic light scattering. The dimension of the particles, as determined with this method varied from 50 nm to 200 nm.

The data obtained on the manganese-Na mixed clearly point towards the formation of a layered Mn(III) / Mn(IV) mixed oxide, essentially devoid of Mn(II).

**General procedure for the oxidative cleavage:** in a 10 mL flask equipped with a stirring bar are placed: the catalyst (1 mol% of Mn), meso-hydrobenzoin (42.9 mg, 0.2 mmol) and 1 mL of solvent. 20 µL of n-dodecane is added as internal standard. Additives, such as a base may be added when required. The reaction medium is heated at a pre-determined temperature in an oil bath and stirred at 400 rpm under an atmospheric pressure of pure O₂ for 1 hour. After this time, the reaction medium is centrifuged, filtered and the filtrate analyzed by gas chromatography. Conversion and selectivity are determined by integration of the area of the peaks and n-dodecane and compared with a calibration curve.

### Example 3: Screening of the catalysts:

Materials prepared according to example 1 and 2 were first tested in the oxidative cleavage of meso-hydrobenzoin. The results are shown in table 3:

**Table 3**

| Catalyst | Yield at 1 h | Selectivity |
|---|---|---|
| MnO2-Na | 98 | 100 |
| MnO2-Ca | 80 | 100 |
| MnO2-Fe | 34 | 100 |
| MnO2-Mg | 19 | 100 |
| MnO2-Sr | 93 | 100 |
| MnO2-Zn | 93 | 100 |

It can be seen from these results that the Manganese-Na mixed oxide is the most efficient in the oxidative cleavage of meso-hydrobenzoin. The other types of Manganese-X mixed oxides are also efficient in this reaction, providing benzaldehyde with good yields and selectivity in only 1 hour.

### Example 4: Compared activities of the Manganese-Na mixed oxide according to example 1 with commercial activated MnO2 and birnessites according to prior art:

Although the prepared catalysts possess common features with birnessite, the method for their preparation results in the formation of a different product.

The activity of the materials according to example 1 has been compared with the one of commercial MnO₂, activated or not and a synthetic birnessite prepared according to Chemical Communications, 1996, 1607. The results are summarized in table 4.

**Table 4**

| **Catalyst** | **Yield** | **Selectivity** |
|---|---|---|
| 1 mol% Mn-Na according to example 1 | 99 | 100 |
| 1 mol% Birnessite according to Chemical Communications, 1996, 1607 | 33 | 100 |
| Commercial activated MnO₂ 1 mol% | 5 | 100 |
| Commercial MnO₂ 1 mol% | 5 | 100 |
| 100 ppm Mn-Na according to example 1 | 70* | 100 |

| | | |
|---|---|---|
| * TON = 180; TOF = 3 min⁻¹ | | |

These results confirm that birnessites are active catalyst in oxidative cleavage reactions but also that the material according to the present invention, obtained by addition of an hydroxide to a mixture of manganese salt and an oxidant, is more active. MnO₂ either activated or not only results in the formation of low amounts of benzaldehyde. Reducing the amount of catalyst according to the present invention still affords good yields in 1 hour.

The higher activity of the catalyst according to the present invention may be explained by the process used for the preparation of the catalyst which results in a different, more active material.

### Example 5: improving Mn-Na catalyst activity:

The conditions for the preparation of the catalyst (according to the protocol described in example 1) were studied. The corresponding catalysts have then been tested in the oxidative cleavage of meso-hydrobenzoin (table 5).

**Table 5**

| **Equivalent HO per Mn (nature of the hydroxide)** | **Conversion** | **Selectivity(%)** |
|---|---|---|
| 4 (NaOH) | 84 | 100 |
| 20 (NaOH) | 93 | 100 |
| 42 (NaOH) | 99 | 100 |
| 52 (NaOH) | 14 | 100 |
| 60 (NaOH) | 18 | 100 |
| 42 (CsOH) | 15 | 100 |
| 42 (Me₄NOH) | 47 | 100 |

As can be derived from the results in table 5, the most efficient catalyst is obtained when NaOH is used in a ratio of 42:1 with respect to the manganese salt.

Other hydroxides in the same proportion, lead to the formation of active, yet less efficient catalysts.

### Example 6: Varying the substrate

Using the protocol of example 3 but varying the substrate, the following assays have been performed:

| **Substrate** | **Amount of Mn-Na according to example 1 (mol%)** | **Yield** | **Time (h)** | **Comment** |
|---|---|---|---|---|
| 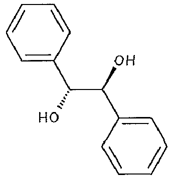 | 10 | 100 | 1 | |
| 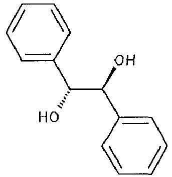 | 1 | 100 | 1 | |
| 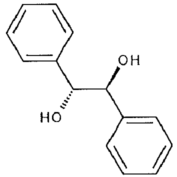 | 1 | 99 | 4 | Air as oxidant instead of O₂ |
| 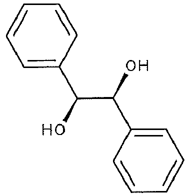 | 10 | 93 | 1 | |
| 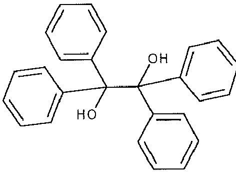 | 1 | 100 | 1 | |
| 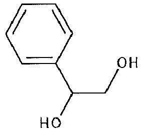 | 10 | 15 | 19 | No base |
| 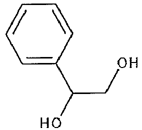 | 10 | 49 | 19 | 2 eq. K₂CO₃ |
| 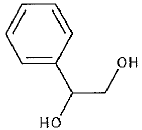 | 10 | 43 | 19 | 0.1 eq. Na₂CO₃ |
| 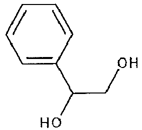 | 10 | 53 | 19 | 2 eq. Na₂CO₃ |
| 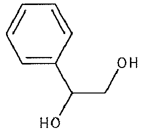 | 10 | 35 | 19 | 0.1 eq. KOH |
| 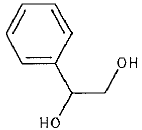 | 10 | 32 | 19 | 2 eq. K₃PO₄ |
| 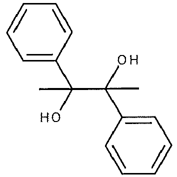 | 10 | 92 | 4 | |
| 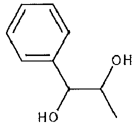 | 10 | 94 | 6 | |
| 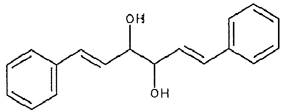 | 10 | 97 | 1 | |
| 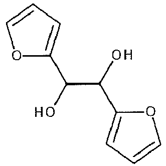 | 10 | 94 | 4 | |
| 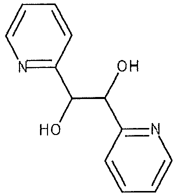 | 1 | 99 | 1 | |
| 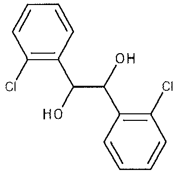 | 10 | 100 | 1 | |
| 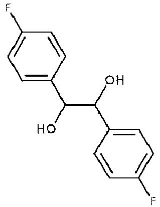 | 10 | 100 | 1 | |
| 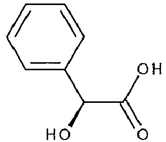 | 10 | 17 (2h) | 2 | Ethanol 80 °C, No base |
| | | 80 (27h) | 27 | |
| 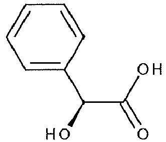 | 10 | 68 (2h) | 2 | Ethanol 80 °C 2 eq. Na₂CO₃ |
| | | 83 (7h) | 7 | |

The results obtained show that the Manganese-Na mixed oxide according to the present invention is highly active for the oxidative cleavage of 1,2-diols and α-hydroxyacids. With some substrates, the addition of a base enables a significative yield improvement.

## Claims

1. Use of a manganese-X mixed oxide, wherein the manganese is at the oxidation state +III and +IV and X is a cation selected from the group consisting of sodium, calcium, magnesium, cesium, lithium, potassium, iron, zinc, strontium, cobalt, cerium, vanadium, molybdenum, tin, tungsten, titanium, chromium, nickel, copper, ruthenium, bismuth, antimony, aluminum, silver, rhenium, osmium, rhodium, iridium, palladium, gold and tetramethylammonium for the oxidative cleavage of a 1,2-diol; a 1,2-diol monoether, a 1,2-diamine; a 1,2-aminoalcohol; an α-hydroxy-carbonyl compound, in particular selected from an α-hydroxy-acid or a derivative thereof such as an α-hydroxy-ester, an α-hydroxy-ketone and an α-hydroxy-aldehyde; an α-dicarbonyl compound such as an α-ketoaldehyde, α-diketone or glyoxal; or an epoxide; preferably a 1,2-diol or an α-hydroxy-acid.

2. Use according to claim 1, **characterized in that** the manganese mixed oxide is a natural or synthetic birnessite or buserite.

3. Use according to any of claims 1 or 2, **characterized in that**, in the 1,2-diol; 1,2-diamine; 1,2-aminoalcohol; α-hydroxy-carbonyl compound, α-dicarbonyl compound or epoxide:
• at least one of the hydroxyl groups, in particular the two hydroxyl groups of the diol, is adjacent to a multiple bond, such as a double or a triple bond, an aromatic ring or a heteroaromatic ring,
• at least one of the amino groups of the 1,2-diamine, in particular the two amino groups of the diamine, is adjacent to a multiple bond, such as a double or a triple bond, an aromatic ring or a heteroaromatic ring, or
• the amino group or the hydroxyl group, in particular both the amino group and the hydroxyl group of the 1,2-aminoalcohol; is adjacent to a multiple bond, such as a double or a triple bond, an aromatic ring or a heteroaromatic ring, or
• the hydroxyl of the α-hydroxy-carbonyl compound is adjacent to a multiple bond, such as a double or a triple bond, an aromatic ring or a heteroaromatic ring, or
• at least one of the carbonyl group of the α-dicarbonyl compound is adjacent to a multiple bond, such as a double or a triple bond, an aromatic ring or a heteroaromatic ring, or
• the oxygen atom of the epoxide is bound to at least one carbon atom, in particular to two carbon atoms adjacent to a multiple bond, such as a double or a triple bond, an aromatic ring or a heteroaromatic ring.
• an α-dicarbonyl compound wherein at least one of the carbonyl groups is adjacent to a multiple bond, such as a double or a triple bond, an aromatic ring or a heteroaromatic ring.

4. A process for preparing a carbonyl derivative, comprising the step of bringing into contact a 1,2-diol, a 1,2-diol monoether, a 1,2-diamine, a 1,2-aminoalcohol or an α-hydroxy-carbonyl compound in particular selected from an α-hydroxy-acid or a derivative thereof such as an α-hydroxy-ester, an α-hydroxy-ketone and an α-hydroxy-aldehyde, an α-dicarbonyl compound such as an α-ketoaldehyde, α-diketone or glyoxal; or an epoxide, preferably a 1,2-diol or an α-hydroxy-acid with a manganese-X mixed oxide as defined in any of claims 1 to 3 and an oxidant, preferably a 1,2-diol or an α-hydroxy-carbonyl compound.

5. The process according to claim 4, wherein the 1,2-diol, 1,2-diol monoether, 1,2-diamine or 1,2-aminoalcohol has formula (I): 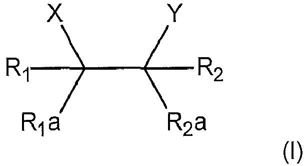 or the epoxide has formula (Ia): 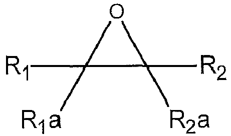 wherein:
X and Y, identical or different, are selected from OH, OR₃ and NHR₄, R₁, R₂, R₁ₐ and R₂ₐ, identical or different, are selected from the group consisting of H, alkyl, alkenyl, alkynyl, aryl, heteroaryl, cycloalkyl, heterocycloalkyl, alkyl-aryl, alkenyl-aryl, alkynyl-aryl, alkyl-heteroaryl, alkenyl-heteroaryl and alkynyl-heteroaryl. R₃ is selected from the group consisting of alkyl, cycloalkyl, heterocycloalkyl, alkyl-aryl and alkyl-heteroaryl.
R₄ is selected from the group consisting of H, alkyl, aryl, heteroaryl, cycloalkyl, heterocycloalkyl, alkyl-aryl, alkenyl-aryl, alkynyl-aryl, alkyl-heteroaryl, alkenyl-heteroaryl and alkynyl-heteroaryl,
to give a first carbonyl derivative of formula (II): 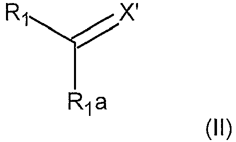 and a second carbonyl derivative of formula (III): 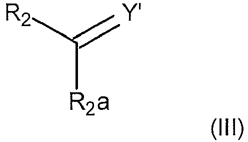
wherein R₁, R₂, R₁ₐ et R₂ₐ are as defined in formula (I), and
X' and Y', identical or different, represent O or NR₄
or
wherein the α-hydroxy-carbonyl compound has formula (IV): 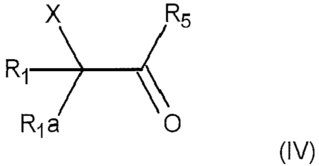 wherein:
R₁ and R₁ₐ, identical or different, are selected from the group consisting of H, alkyl, alkenyl, alkynyl, aryl, heteroaryl, cycloalkyl, heterocycloalkyl, alkyl-aryl, alkenyl-aryl, alkynyl-aryl, alkyl-heteroaryl, alkenyl-heteroaryl and alkynyl-heteroaryl,
X is selected from OH and NHR₃,
R₅ is selected from the group consisting of H, OH, OR_{b}, NR_{c}R_{d}, alkyl, alkenyl, alkynyl, aryl, heteroaryl, cycloalkyl, heterocycloalkyl, alkyl-aryl, alkenyl-aryl, alkynyl-aryl, alkyl-heteroaryl, alkenyl-heteroaryl and alkynyl-heteroaryl, in which R_{b}, R_{c} and R_{d} are selected from the group consisting of alkyl, alkenyl, alkynyl, aryl, heteroaryl, cycloalkyl, heterocycloalkyl, alkyl-aryl, alkenyl-aryl, alkynyl-aryl, alkyl-heteroaryl, alkenyl-heteroaryl and alkynyl-heteroaryl or R_{c} and R_{d} form together with the nitrogen atom to which they are linked a heterocycloalkyl,
to give a carbonyl derivative of formula (II): 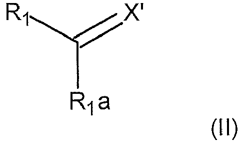
wherein R₁ and R₁ₐ are as defined in formula (IV), and
X' is selected from OH and NHR₄,
or
wherein the α-dicarbonyl compound is an α-ketoaldehyde, an α-diketone, an α-keto-acid or glyoxal having formula (V): 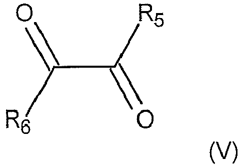 wherein:
R₅ is selected from the group consisting of H, OH, OR_{b}, NR_{c}R_{d}, alkyl, alkenyl, alkynyl, aryl, heteroaryl, cycloalkyl, heterocycloalkyl, alkyl-aryl, alkenyl-aryl, alkynyl-aryl, alkyl-heteroaryl, alkenyl-heteroaryl and alkynyl-heteroaryl, in which R_{b}, R_{c} and R_{d} are selected from the group consisting of alkyl, alkenyl, alkynyl, aryl, heteroaryl, cycloalkyl, heterocycloalkyl, alkyl-aryl, alkenyl-aryl, alkynyl-aryl, alkyl-heteroaryl, alkenyl-heteroaryl and alkynyl-heteroaryl or R_{c} and R_{d} form together with the nitrogen atom to which they are linked a heterocycloalkyl,
R₆ is selected from the group consisting of H, alkyl, alkenyl, alkynyl, aryl, heteroaryl, cycloalkyl, heterocycloalkyl, alkyl-aryl, alkenyl-aryl, alkynyl-aryl, alkyl-heteroaryl, alkenyl-heteroaryl and alkynyl-heteroaryl,
to give a carboxylic acid of formula (VII): 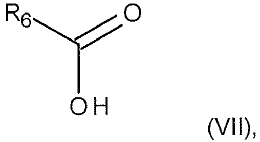 wherein R₆ is as defined in formula (V).

6. The process according to claim 7, wherein at least one of R₁, R₂, R₁ₐ et R₂ₐ is selected from the group consisting of: aryl, heteroaryl,

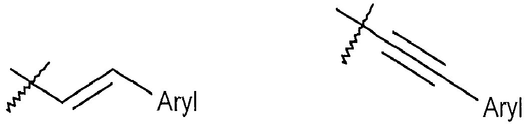

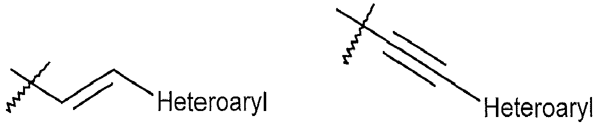

7. The process according to claim 6, **characterized in that** the oxidant is pure dioxygen or air.

8. The process according to claim 6 or 7, wherein the compound of formula (I) is a diol selected from the group consisting of: 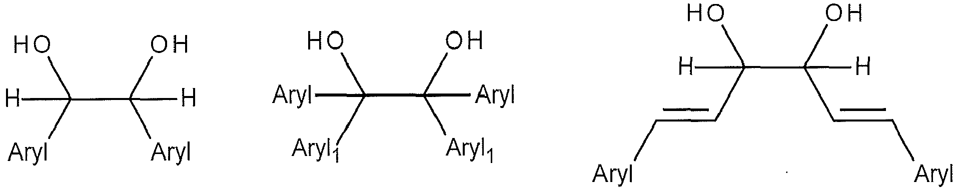 or an α-hydroxy-acid of the following formula (Va1): 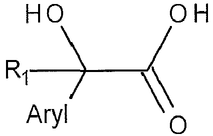 wherein the aryl is in particular an optionally substituted phenyl.

9. Process for preparing a Manganese-X mixed oxide, wherein the manganese is at the oxidation state +III and +IV and X is a cation selected from the group consisting of sodium, calcium, magnesium, cesium, lithium, potassium, iron, zinc, strontium, cobalt, cerium, vanadium, molybdenum, tin, tungsten, titanium, chromium, nickel, copper, ruthenium, bismuth, antimony, aluminum, silver, rhenium, osmium, rhodium, iridium, palladium, gold and tetramethylammonium, **characterized in that**:
(a) an hydroxide of formula Xⁿ⁺(HO⁻)ₙ is added to a solution containing a Manganese (II) salt and an oxidant, or
(b) an hydroxide of formula M^{m+}(HO⁻)ₘ is added to a solution containing a manganese (II) salt, an oxidant and a compound of formula Xⁿ⁺(Y^{p-})_{n/p} in which M is a metal selected in particular from Li, K and Na and Y is a neutral counterion such as a chloride or a sulfate.

10. The process according to claim 9, **characterized in that** the hydroxide is added at a proportion of 42:1, molar with respect to the manganese (II) salt.

11. The process according to any one of claims 15 to 20, **characterized in that** :
a. the manganese salt is preferably manganese chloride,
b. X is sodium and the hydroxide is sodium hydroxide, in proportion of 4:1 to 50:1, in particular 42:1, molar with respect to the manganese (II) salt,
c. the oxidant is hydrogen peroxide, in proportion 3:1, molar with respect to the manganese (II) salt.

12. A Manganese-X mixed oxide, wherein the manganese is at the oxidation state +III and +IV and X is sodium, **characterized in that** manganese is present in proportion of 45 to 55 % and the sodium in proportion of 1 to 10 %, by weight of the total weight of said mixed oxide.

13. The Manganese-X mixed oxide according to claim 13, wherein the manganese is at the oxidation state +III and +IV and X is sodium, **characterized in that** it is obtainable by a process comprising the step of adding NaOH in proportion of 4:1 to 50:1, in particular 42:1, molar with respect to the manganese (II) salt to a solution containing a Manganese (II) salt, preferably MnCl₂, and an oxidant, preferably hydrogen peroxide, in particular in proportion of 3:1, molar with respect to the manganese (II) salt.

14. The manganese-X mixed oxide according to any of claims 9 to 13, **characterized in that** it is devoid of Manganese at the state of oxidation +2.

15. Use of a Manganese-X mixed oxide according to any one of claims 9 to 14, in particular according to claim 13, as an oxidation catalyst, in particular for the oxidative cleavage of a 1,2-diol, 1,2-diol monoether, a 1,2-diamine, a 1,2-aminoalcohol or an α-hydroxy-carbonyl compound, in particular selected from an α-hydroxy-acid or a derivative thereof such as an α-hydroxy-ester, an α-hydroxy-ketone and an α-hydroxy-aldehyde an α-dicarbonyl compound such as an α-ketoaldehyde, α-diketone or glyoxal; or an epoxide, preferably a 1,2-diol or an α-hydroxy-acid, the oxidation of sulfides into sulfoxides or sulfones, the formation of azo compounds from anilines, the C-H oxidation of aromatic rings and the oxidation of alcohols, in particular of allylic, benzylic and propargylic alcohols.
